Europäisches Patentamt

European Patent Office

Office européen des brevets

(11) Numéro de publication: **0 312 668**
**A1**

## (12) DEMANDE DE BREVET EUROPEEN

(21) Numéro de dépôt: 87402618.0

(22) Date de dépôt: 22.10.87

(51) Int. Cl.⁴ **A61K 7/50**

(43) Date de publication de la demande:
26.04.89 Bulletin 89/17

(84) Etats contractants désignés:
ES GR

(71) Demandeur: CHRY Sarl
Penvigne
F-49400 Villebernier(FR)

(72) Inventeur: Ruffay, Marc
28, rue Lantiez
F-75017 Paris(FR)

(74) Mandataire: Le Guen, Louis François
Cabinet Louis Le Guen 38, rue Levavasseur
B.P. 91
F-35802 Dinard Cédex(FR)

(54) **Bain moussant effervescent.**

(57) La composition du bain moussant sous forme solide à pouvoir effervescent est la suivante:
lauryl sulfate de sodium de 24 à 34 %
monoéthanolamide d'acide gras de coprah de 4 à 14 %
bicarbonate de soude de 28 à 38 %
acide citrique de 20 à 30 %
parfum hypoallergique, principe actif
huile de vison, principe actif
colorant, principe actif
excipient de compression QS pour un comprimé de 7 g contenant 7 % de produits actifs.

EP 0 312 668 A1

## Bain moussant effervescent

La présente invention concerne la composition d'un bain moussant sous forme solide et possédant un pouvoir effervescent.

Les bains moussants déjà existants sont soit sous forme liquide, soit sous forme solide (sels de bain), et doivent être employés en quantité relativement importante pour obtenir un effet moussant.

La composition selon l'invention permet de pallier à cet inconvénient. A cet effet, elle comprend une base moussante hautement concentrée sous forme de lauryl sulfate de sodium dont le poids n'excède pas 25 g par bain moussant. Celui-ci est rapidement solubilisé par action d'effervescence.

L'action moussante est provoquée par l'introduction du lauryl sulfate de sodium et d'un stabiliseur de mousse tel que le monoéthanolamide d'acide gras de coprah. L'effervescence est obtenue par la réaction entre le bicarbonate de soude et l'acide citrique, par exemple. L'ensemble de la composition est présentée sous forme de comprimé compact.

Plus particulièrement, la composition du bain moussant peut être la suivante:
lauryl sulfate de sodium de 24 à 34 %
monoéthanolamide d'acide gras de coprah de 4 à 14 %
bicarbonate de soude de 28 à 38 %
acide citrique de 20 à 30 %
parfum hypoallergique, principe actif
huile de vison, principe actif
colorant, principe actif
excipient de compression QS pour un comprimé de 7 g contenant 7 % de produits actifs

Un exemple préféré de composition est le suivant:
lauryl sulfate de sodium, 29 %
monoéthanolamide d'acide gras de coprah, 9 %
bicarbonate de soude, 33 %
acide citrique, 25 %
parfum hypoallergique, 3 %
huile de vison, 0,5 %
colorant 0,5 %
excipient de compression QS pour comprimé de 7 g contenant 7 % de produits actifs

Le composé lauryl sulfate de sodium se trouve dans le commerce sous le nom commercial de SIPON LCSV95 et le monoéthanolamide sous le nom commercial de COMPERLAN 100

Un comprimé de 7 g de cette composition donne une dose unitaire pour une baignoire de 200 l environ.

## Revendications

1) Composition d'un bain moussant sous forme solide et possédant un pouvoir effervescent caractérisée en ce qu'elle a la composition suivante:
lauryl sulfate de sodium de 24 à 34 %
monoéthanolamide d'acide gras de coprah de 4 à 14 %
bicarbonate de soude de 28 à 38 %
acide citrique de 20 à 30 %
parfum hypoallergique, principe actif
huile de vison, principe actif
colorant, principe actif
excipient de compression QS pour un comprimé de 7 g contenant 7 % de produits actifs

2) Composition suivant la revendication 1, caractérisée en ce qu'elle a la composition suivante:
lauryl sulfate de sodium, 29 %
monoéthanolamide d'acide gras de coprah, 9 %
bicarbonate de soude, 33 %
acide citrique, 25 %
parfum hypoallergique, 3 %
huile de vison, 0,5 %
colorant 0,5 %
excipient de compression QS pour comprimé de 7 g contenant 7 % de produits actifs

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| E | FR-A-2 597 338 (RUFFRAY) <br> * En entier * <br> --- | 1,2 | A 61 K 7/50 |
| Y | FR-A-2 549 723 (GREFF) <br> * En entier * <br> --- | 1,2 | |
| Y | US-A-3 489 688 (POSPISCHIL) <br> * En entier * <br> --- | 1,2 | |
| Y | WO-A-8 602 832 (UNION CARBIDE) <br> * Page 10, ligne 6 - page 11, ligne 19; page 13, ligne 28 - page 16, ligne 10; revendications 1,4-7,9,10,14 * <br> --- | 1,2 | |
| Y | FR-A-2 152 810 (THE GILLETTE CO.) <br> * En entier * <br> ----- | 1,2 | |

DOMAINES TECHNIQUES
RECHERCHES (Int. Cl.4)

A 61 K

Le présent rapport a été établi pour toutes les revendications

| Lieu de la recherche | Date d'achèvement de la recherche | Examinateur |
|---|---|---|
| LA HAYE | 06-07-1988 | FISCHER J.P. |